# FASCICULE DE BREVET EUROPEEN

(11) **EP 0 732 927 B1**
(45) Date de publication et mention de la délivrance du brevet: **30.10.2002**
(21) Numéro de dépôt: 95916739.6
(22) Date de dépôt: 11.04.1995
(51) Int. Cl.: A61K 31/525

(54) **COMPOSITIONS D'ENTRE AUTRES LA RIBOFLAVINE POUR LA PROTECTION LOCALE DES MUQUEUSES GENITALES ET RECTALES**
ZUSAMMENSETZUNGEN MIT U.A. RIBOFLAVIN ZUR LOKALER SCHÜTZUNG VON GENITALEN UND RECTALEN SCHLEIMHÄUTEN
COMPOSITIONS CONTAINING IN PARTICULAR, RIBOFLAVIN, FOR THE LOCAL PREVENTION OF DISEASES OF THE GENITAL AND RECTAL MUCUS MEMBRANES

(30) Priorité: 11.04.1994 FR 9404232
(43) Date de publication de la demande: 25.09.1996
(73) Titulaire: BERQUE, Jean, F-16100 Cognac (FR)
(72) Inventeur: BERQUE, Jean, F-16100 Cognac (FR)
(74) Mandataire: Burtin, Jean-François
(86) Numéro de dépôt international: FR9500463
(87) Numéro de publication internationale: WO95027491

(56) Documents cités:
- EP-A- 0 530 862
- WO-A-92/01439
- US-A- 5 075 116
- DATABASE WPI Week 8817 Derwent Publications Ltd., London, GB; AN 88-115373 & JP,A,63 060 910 (SHISEIDO) , 17 Mars 1988

## Description

Dans sa demande de brevet français n° 92.12180, déposée le 2 Octobre 1992 et délivrée sous le n° 2.696.319, le demandeur a déjà décrit l'utilisation de la riboflavine dans la fabrication des préservatifs et des gants de protection médico-chirurgicaux.

L'utilisation des préservatifs en vue de la prévention des maladies sexuellement transmissibles et notamment des maladies à virus HSV2, HIV ainsi que les infections génito-anales à papillomavirus ne paraît pas obéir aux recommandations sanitaires prophylactiques. Cette restriction d'usage en rapport avec des motifs socio-économiques, techniques ou sexuels amène à proposer d'autres solutions afin de limiter la propagation et le contage vénériens. L'utilisation de pommade, ovules et suppositoires composés de molécules antivirales biologiques et atoxiques paraît répondre à ce besoin. Une telle alternative semble d'autant plus avantageuse que les molécules utilisées peuvent, en outre, présenter des propriétés anti-allergiques, anti-inflammatoires, anti-bactériennes, antifongiques, immunostimulantes et cicatrisantes. Des essais avec des molécules chimiques de synthèse antivirales ont été effectués.

C'est ainsi qu'ont été proposées des éponges à base de nonoxynol-9 dans un but à la fois contraceptif et virulicide. Ces essais sont loin d'être concluants sur le plan de l'efficacité et de surcroît les produits utilisés généreraient des micro-lésions de la paroi vaginale. Une fois de plus, l'expérimentation apporte la preuve du fossé existant entre l'efficacité "in vitro" puis "in vivo" et la tolérance.

L'invention a donc pour objet l'utilisation d'une composition à base de molécules biologiques ayant montré une activité antivirale « in vitro » mais totalement atoxique et même présentant des propriétés curatives locales par rétablissement de la chaîne respiratoire cellulaire, de l'équilibre d'oxydoréduction et la cicatrisation de micro-lésions muqueuses d'origine carentielle.

En conséquence, l'invention a pour objet l'utilisation de la Riboflavine ou d'un de ses sels, en vue de la réalisation d'un médicament antiviral à effet local, destiné au traitement des muqueuses génitales et rectales.

L'invention a également pour objet l'utilisation aux mêmes fins du FAD.

Le principe actif peut être additionné en outre par de la Niacine et/ou du NAD et/ou du NADP. On peut y ajouter également des facteurs vitaminiques tels que les carotènes, les flavonoïdes, l'acétylcoenzyme A, et les vitamines A, E, C, B6, B8 et/ou F, ainsi que de l'acide folique (Vitamine B₉). Les facteurs vitaminiques peuvent se présenter sous forme de base et/ou sous leur forme native.

On peut également ajouter au médicament antiviral selon l'invention des agents de charge qui renforcent l'adhésivité des préparations aux muqueuses comme des sels insolubles de baryum, en particulier le sulfate de baryum ou le titanate de baryum ; ou des dérivés du titane choisis parmi le dioxyde de titane, le chlorure de titane ou le mica-titane.

Le médicament antiviral selon l'invention peut en outre contenir du lysozime.

On peut, en outre, incorporer aux compositions pharmaceutiques selon l'invention des extraits végétaux contenant des flavonoïdes ou des anthrocyanosides.

On peut encore incorporer aux compositions pharmaceutiques du glutathium et/ou de la N-acétylcystéine.

Les formes pharmaceutiques sont celles appropriées pour la protection de la sphère génitale ou pour la protection de la muqueuse rectale, comme les crèmes, les gels, les laits, les émulsions visqueuses, les suppositoires, les ovules, les capsules, les comprimés vaginaux et les formes semblables.

L'effet initial revendiqué concerne avant tout la prévention des maladies virales transmissibles sexuellement (Sida, herpès génital et papillomavirus). Néanmoins, si le même mélange peut à la fois être curatif et présenter des propriétés complémentaires, il s'agit d'un avantagé indéniable. Ce seront essentiellement des effets antibactériens, antifongiques, antiparasitaires, anti-allergiques et anti-inflammatoires, trophiques et cicatrisants et enfin, vasculotropes.

La riboflavine et le FAD (ou le FADH2) peuvent être utilisés isolément. Toutefois, la complémentarité et la synergie d'action apportées par la niacine et ses formes actives, l'acétylcoenzyme A, les vitamines A, E, C, la biotine, les flavonoïdes, les carotènes sont intéressantes à considérer. L'adjonction du pyridoxal-5-phosphate et de N5-méthyltétrahydrofolate paraît utile.

Une telle composition à polarité anti-oxydante est destinée à être appliquée ou introduite au niveau des muqueuses en fonction des connaissances de la pharmacocinétique et de la galénique.

Les préparations destinées à l'application locale pourront se présenter sous forme de pommade, gel, crème, mousse ou toute autre préparation issue de la galénique moderne. Les préparations destinées à l'introduction endo-vaginale ou endo-rectale, seront de constitution analogue aux précédentes mais pourront également être véhiculées par des ovules, suppositoires, éponges, gélules, comprimés ou tout autre réservoir à micro-diffusion.

Quelle que soit la forme utilisée, le but essentiel sera de présenter un rapport efficacité/tolérance optimal. Elle devra donc allier des pouvoirs couvrants, antioxydants et antiviraux; des effets complémentaires trophiques, cicatrisants et immunostimulants voire antifongiques et anti-bactériens constitueront des avantages non négligeables.

Le pouvoir couvrant est intéressant à considérer selon un double aspect : il est tout d'abord susceptible de constituer une barrière matérielle s'opposant à la diffusion des virus et des protéines que ceux-ci synthétisent. Il peut ensuite jouer un rôle de filtre-écran électromagnétique qui bloquerait à la fois UVC, RX et IR.

Selon le demandeur, la double hélice ADN des cellules et des rétrovirus serait la concrétisation du double tore généré par les mouvements oscillants synchrones du champ électrique et du champ magnétique qui accompagnent la lumière et sont perpendiculaires l'un à l'autre. Une telle structure, à double filament ADN inclus dans un noyau dont la membrane est caractérisée par la présence de pores, est analogue à une triode amplificatrice absorbant des ultraviolets et réémettant des infrarouges.

Le fait de bloquer les UVC dont la longueur d'onde correspond aux dimensions virales et les IR réémis empêcherait l'activation voire la synthèse virale (UVC), puis la transmission d'informations virales à distance (IR).

Il paraît, en outre, très intéressant de prévoir l'incorporation, au niveau de la composition proposée, d'éléments opaques comme le baryum et le titane. Les filtres au baryum laissent passer les UVA et les UVB mais absorbent les UVC et les rayons X. C'est pour cette raison que le sulfate de baryum composée de particules de la taille de 1 micron présente un grand pouvoir couvrant et tapisse les moindres replis de la muqueuse.

Selon le demandeur, le baryum représente un complément de choix de la riboflavine. Les composés du baryum émettent à la flamme une coloration jaune-vert. Cette coloration est complémentaire des longueurs d'onde correspondant au rouge et de celles immédiatement supérieures qui correspondent à l'infrarouge. Le baryum qui est peu toxique et qui possède un grand pouvoir couvrant, arrête les UVC et les IR. Il paraît représenter un élément de choix dans la protection locale des muqueuses contre les virus. A titre d'exempte, le titanate de baryum est un isolant des plus performants. L'association de titane et de baryum à la riboflavine apparaît très avantageuse pour plusieurs raisons Elle protège tout d'abord les membranes contre les rayonnements extrêmes, pathogènes, et en particulier les ultraviolets de haute énergie (UVB et UVC) et les infrarouges les plus fréquentiels (1,5 à 0,8 micron). Le demandeur a déjà émis l'hypothèse selon laquelle les virus peuvent, du fait de leurs dimensions, entrer en phase avec les UV et notamment les UVC, réalisant ainsi une résonance parfaite. Une oxydation exagérée en rapport avec des UVC serait activatrice virale; inversement des processus de fermentation exagérée couplés avec un rebond infrarouge excessif stimuleraient la croissance bactérienne et mycologique. Ainsi donc, l'association titane-baryum-riboflavine constitue un écran aux radiations extrêmes UV et IR et se comporte à la fois comme un inhibiteur viral, bactérien, fongique et parasitaire. Une telle ssociation est en même temps anti-allergique.

Le mélange précédent est en outre anti-inflammatoire en raison de ses propriétés anti-oxydantes associées à celles déjà décrites. Le deuxième intérêt du complexe titane-baryum-riboflavine concerne les membranes cellulaires mitochondriales à cause de leur structure en bi-couche lamellaire. Le titane et le baryum vont doper le semi-conducteur membranaire et catalyser énergiquement le transfert de charges entre les cytochromes, porteurs des électrons et de l'oxygène, et de la pompe à protons. A l'étage du système immunitaire, il constitue ainsi un amplificateur de la synthèse des anticorps. Il peut donc être considéré comme un immunostimulant.

Le titane intégré au complexe antioxydant et photoprotecteur pourra être utilisé au moins sous trois formes : dioxyde de titane, chlorure de titane et mica-titane. La forme chlorure présente l'intérêt, en outre, de freiner les infrarouges. L'extrapolation de ces qualités au niveau des molécules photo-sensibles antivirales et plus particulièrement de la riboflavine apparaît des plus intéressantes. Le chlore et les halogènes saturent au niveau des acides gras insaturés les doubles liaisons éthyléniques et modulent ainsi la bêta-oxydation lipidique.

Selon le demandeur, la beta-oxydation, alternativement activée par Ca++ et Mg++, participerait à l'ouverture et à la création des canaux membranaires. Le chlore, en saturant certaines chaînes phospho-lipidiques modulerait par là-même le nombre et la fréquence des ouvertures canalaires. Alors que les UV présentent un tropisme certain pour les doubles liaisons éthyléniques, les graisses saturées constituent un écran au même rayonnement UV. Or, la plupart des éléments et molécules, définis par la présente invention, ont été choisis pour leurs effets antioxydants. Ils jouent presque tous, en fait, un rôle d'oxydo-réducteurs. En effet, si l'oxydation est toxique pour les cellules par suite de la formation de radicaux libres, elle est en général nécessaire. Elle est tout d'abord utile à la création d'un gradient de charges électriques et à la circulation des électrons et des protons. Elle est également indispensable au catabolisme des aliments, à la respiration cellulaire et à la phosphorylation qui sont des processus oxydatifs. Elle jouerait enfin un rôle capital dans la stimulation de certains mécanismes de défense de l'organisme. C'est ainsi que les peroxydes et particulièrement l'oxygène singulet, caractérisé par sa chimie luminescence, sa durée de vie extrêmement brève et sa grande agressivité, détruiraient les cellules pathologiques ayant dépassé un seuil de létalité. L'oxydation stimulerait par ailleurs au niveau des leucocytes et notamment des macrophages une enzyme dont le rôle paraît spécialement dévolu à la destruction de micro-organismes agresseurs.

La NADPH oxydase est une flavo-enzyme B2 et PP dépendantes.

La destruction des agents pathogènes comme les bactéries et les virus peut d'autres fois, obéir à des procédés biochimiques et notamment la production au niveau des leucocytes, d'hypochlorite OCl⁻. Les antioxydants ont la particularité de transformer les peroxydes en hydroperoxydes à durée de vie beaucoup plus longue et nettement moins pathogènes.

Le mica-titane est utilisé dans les crèmes solaires pour ses propriétés photo-réflectrices. Le mica est un silicate d'aluminium. A l'instar du titane et du baryum, l'aluminium et le silicium sont étroitement complémentaires. Ils constituent en parallèle un système semi-conducteur, semi-filtrant et piezo-électrique qui fait alternativement office d'écran et d'isolant puis de conducteur optoélectronique.

Au niveau des semi-conducteurs et transistors membranaires, le tandem silice-aluminium joue un rôle équivalent au couple baryum-titane. Il arrête les impulsions optoélectroniques extrêmes, pathogènes, et catalyse "a contrario" le transfert des énergies adéquates. Il constitue ainsi un antioxydant et en même temps un dopant des échanges respiratoires. Ce mécanisme d'action explique pour partie les vertus trophiques et cicatrisantes du silicium et de l'aluminium sur la peau et les muqueuses. L'aluminium est dans un premier temps très sensible à l'oxydation, mais il se recouvre rapidement d'une pellicule d'hydroxyde d'alumine très résistante. De telles propriétés sont fondamentales au niveau de la composition anti-oxydante revendiquées en particulier pour ses qualités antivirales et anti-méthémoglobinisantes. D'autres métaux et métalloïdes ont un intérêt certain en raison de leurs effets antioxydants, catalytiques (activateurs enzymatiques et/ou dopants membranaires) ainsi que leur rôle de filtre-écran optoélectronique et modulateur fréquentiel.

Selon le demandeur, un taux élevé de LDL cholestérol est le témoin d'une menace auto-immune. Certains auteurs ont émis l'hypothèse selon laquelle les virus et, en particulier, les virus de l'herpès induiraient une athéromatose; "théorie virale de l'athéromatose. Place du cytomégalovirus "J. Byad et Col. Ann. Biol. Clin. (1993) 51.101-107. Selon les hypothèses du demandeur, l'infection virale et l'athéromatose ne seraient pas directement interdépendantes; elles seraient, en fait, deux pathologies contiguës qui témoigneraient du fait que l'organisme est victime d'un stress oxydatif.

La théorie des deux pathologies contiguës paraît corroborée par le fait que la thérapeutique antivirale étudiée par le Demandeur et composée de molécules biologiques photoprotectrices et anti-oxydantes, présente en même temps des effets anti-athéromateux; elle permet aussi de lutter contre le stress oxydatif et l'affection auto-immune responsable.

En ce qui concerne la protection des muqueuses génito-anales, l'effet recherché étant à court terme, il est préférable d'utiliser des graisses saturées conférant à la composition un pouvoir couvrant, photoprotecteur et antioxydant. Ces graisses saturées pourront servir de vecteur aux vitamines liposolubles, c'est-à-dire essentiellement des carotènes, comme les bêta-carotènes et la cantaxanthine, de la vitamine A sous forme aldéhyde ou acide et de l'acétate d'alpha-tocophérol (vitamine E). Les graisses saturées pourront consister en des acides gras linéaires comportant plus de 8 atomes de carbone, comme par exemple l'acide palmitique (C16), l'acide arachidique (C20) et l'acide cérotique (C26) ou en des acides gras ramifiés dont les esters de la lanoline et la lanoline elle-même. Ce pourrait-être des précurseurs du cholestérol comme les squalènes qui seront éventuellement saturés par hydrogénation ou halogénation, le lanostérol riche en groupements méthyles et enfin, le cholestérol lui-même.

Au total, les produits actifs qui peuvent être incorporés à la composition revendiquée comprennent : les vitamines B2, PP, B6, B8, B9, A, E, C, les carotènes (bêta-carotènes, cantaxanthine), l'acétylcoenzyme A et les flavonoides. Dans le cas où les conditions métaboliques locales ne permettraient pas la transformation des formes de base en formes actives, il paraît souhaitable de délivrer celles-ci directement sous leur forme oxydée ou de préférence leur forme réduite. Le FAD et surtout le FADH2 pourraient complémenter la riboflavine; le NAD, le NADH2, le NADP et le NADPH2 complèmenteraient la niacine; le pyridoxal-5-phosphate et le N5-méthyltétrahydrofolate seraient, de la même manière, préférables à la pyridoxine et à l'acide folique.

Des acides aminés comme la cystéine ou la N-acétylcystéine et des peptides comme le glutathion et le lysozyme présentent un intérêt de complémentarité.

Des acides gras saturés à chaîne linéaire (acide palmitique, arachidique, cérotique), ramifiée (lanoline et esters de la lanoline) ou cyclique (squalènes, lanostérol, cholestérol) sont intéressants pour leurs propriétés photoprotectrices et leur rôle de vecteurs des vitamines liposolubles.

Les métaux qui apparaissent comme les plus complémentaires des principes actifs déjà cités sont des sels de baryum (sulfate, titanate), les dérivés du titane (chlorure, dioxyde et mica-titane), les dérivés du silicium et les dérivés de l'aluminium (silicate d'aluminium, hydroxyde d'alumine), le chlore et le fluor, le soufre, le manganèse, le zinc, l'oxyde de zinc, ou zinc ionisé catalytique), ou bien encore les dérivés de métaux, l'argent, surtout sous forme d'argent colloïdal, et enfin le calcium et le magnésium.

Les plantes, comme le mélilot, la myrtille et le millepertuis peuvent ne pas apparaître comme indispensables; néanmoins, le millepertuis peut constituer un additif de choix sous forme de sommités fleuries fraîches d'Hypericum perforatum en macération huileuse, ou de son principe actif, l'hypéricine, à condition qu'il ne soit pas trop allergénique.

L'adjonction de lysozyme et de graisses saturées n'est peut-être pas indispensable, mais elle est tout à fait susceptible de potentialiser les effets requis.

Parmi les métaux dont l'addition est prévue, les suivants sont préférés : le baryum, le titane, l'argent, le sélénium, le germanium, le zinc, le chlore et le fluor.

Les posologies utiles de ces différents composés seront d'environ : 10 à 200 mg pour la riboflavine, 300 à 500 mg pour la niacine (elles seront moindres pour les coenzymes actifs), 500 mg à 1 g pour la pyridoxine, 500 mg à 1 g pour la vitamine C, 20 à 100 mg pour l'acide folique, 20 à 100 mg pour l'acétylcoenzyme A, 50 à 100 mg pour la biotine, 20 à 50 mg pour les bêta-carotènes et la cantaxanthine, 25 à 100.000 UI pour la vitamine A, 20 à 100 mg pour la vitamine E (acétate de tocophérol), 20 à 50 mg pour le lysozyme, 100 à 300 mg pour le mélilot, le millepertuis et la myrtille (doses moindres pour des principes actifs comme l'hypericine)

De quelques µg à quelques mg pour les métaux utilisés dans un but d'activation (chlore, sélénium, aluminium), ceux-ci sont employés à la dose de plusieurs centaines de mg, voire de plusieurs grammes lorsqu'ils sont utilisés pour leurs propriétés couvrantes (oxyde de zinc, hydroxyde d'aluminium, sulfate de baryum).

Les différentes formes galéniques dont l'énumération et la description vont suivre, peuvent être utilisées comme adjuvant des préservatifs ou en dehors de leur usage, dans un but à la fois préventif et curatif. Elles devront donc présenter un pouvoir couvrant maximal mais n'entravant pas la diffusion, la biodisponibilité et l'efficacité des principes actifs. Ces derniers seront d'autant plus intéressants qu'ils auront une durée de vie plus longue. L'usage et le suivi des formes galéniques nécessitent par ailleurs des présentations pratiques et dénuées d'inconvénients désagréables (odeur, écoulements).

Le pouvoir couvrant aura pour but non seulement de tapisser le revêtement cutanéo-muqueux afin non seulement de constituer une barrière matérielle, mais encore de constituer un écran vis-à-vis des rayonnements électriques.

Comme il a été vu précédemment, ces rayonnements seraient à la fois activateurs viraux (UVC) et bactériens, fongiques, etc... (IR). Ils transmettraient en outre une émission à distance, c'est-à-dire des informations fréquentielles d'origine hycoprotéique émanant des virus (IR). Un tel écran matériel et électromagnétique sera encore plus intéressant s'il se comporte comme un semi-conducteur et catalyse les échanges respiratoires et métaboliques. Le sulfate de baryum, éventuellement enrichi en titane, paraît tout à fait convenir à cette définition. Il en est de même du silicate d'aluminium. D'autres composés jouent un rôle important comme le pectate d'aluminium, l'oxyde de zinc, le palmitate de rétinol, la lanoline pour leur pouvoir couvrant. Les principes actifs de la composition revendiquée pourront être véhiculés par une phase grasse pour les vitamines liposolubles (carotènes, vitamine A et E et par une phase aqueuse ou hydroalcoolique pour les vitamines hydrosolubles (B2, PP,B6, C..), le lysozyme, le glutathion, les plantes comme le millepertuis, ainsi que certains métaux comme l'argent, le zinc, le manganèse, etc...

L'ensemble pourrait être inclus dans des liposomes actifs entourées d'une mince pellicule résorbable de silicate d'aluminium en cas de lésions muqueuses vaginales. La couche grasse sous-jacente à la pellicule de silice, colorée en jaune orange par les carotènes, la vitamine A et la vitamine E, serait de la même manière polarisée vers les zones malades de polarité électromagnétique inverse. A l'intérieur des liposomes, les vitamines hydrosolubles et particulièrement la riboflavine, seraient protégées de l'oxydation par la vitamine C, le zinc, le manganèse, etc... Le noyau central des liposomes pourrait quant à lui être constitué de silicate de baryum et de titane qui viendraient ainsi constituer en dernier lieu la barrière couvrante.

Les liposomes actifs, précédemment décrits, pourraient constituer l'unité structurale de base d'un gel à usage pénien, vulvo-vaginal ou rectal (tube canule unidose). Ils pourraient également être inclus dans des ovules, gélules ou comprimés à diffusion lente, ainsi que dans des suppositoires.

L'énumération des formes galéniques est fournie à titre d'illustration mais ne limite pas l'invention.

## Revendications

1. Utilisation de la riboflavine en vue de la réalisation d'un médicament antiviral à effet local destinées au traitement des muqueuses génitales et rectales.

2. Utilisation de la riboflavine selon la revendication 1° **caractérisée en ce que** le médicament contient comme principe actif de la riboflavine et/ou du FAD.

3. Utilisation de la riboflavine selon les revendications 1 et 2° **caractérisée en ce que** le médicament contient en plus de la riboflavine et/ou du FAD, de la niacine et/ou du NAD et du NADP.

4. Utilisation de la riboflavine selon les revendications 1 à 3° **caractérisée en ce que** le médicament contient comme adjuvants thérapeutiques d'autres facteurs vitaminiques tels des carotènes, des flavonoïdes, l'acétylcoenzyme A et les vitamines A, E, C, B6, B8 et B9.

5. Utilisation de la riboflavine selon l'une quelconque des revendications 1 à 4° **caractérisée en ce que** les facteurs vitaminiques sont sous forme de base et/ou sous leur forme native.

6. Utilisation de la riboflavine selon l'une quelconque des revendications 1 à 4° **caractérisée en ce que** les médicaments contiennent en outre du lysozyme.

7. Utilisation de la riboflavine selon l'une quelconque des revendications 1 à 4° **caractérisée en ce que** les médicaments contiennent du glutathion et de la N-acétylcystéine.

8. Utilisation de la riboflavine selon l'une des revendications 1 à 4° **caractérisées en ce que** les médicaments contiennent un sel de baryum choisi parmi le sulfate ou le titanate et/ou un dérivé du titane choisi parmi le dioxyde de titane, le chlorure de titane et le mica-titane.

9. Utilisation de la riboflavine selon l'une des revendications précédentes **caractérisées en ce qu'**elles sont présentées sous les différentes formes galéniques appropriées pour la protection de la sphère génitale.

10. Utilisation de la riboflavine selon l'une des revendications précédentes **caractérisées en ce qu'**elles sont présentées sous les différentes formes galéniques appropriées pour la protection de la muqueuse rectale.

## Patentansprüche

1. Verwendung von Riboflavin für die Herstellung eines viralhemmendes Arzneimittel mit einer ortlichen Wirkung, für die Behandlung von genitalen und rektalen Schleimhäute.

2. Verwendung von Riboflavin nach Ansprüch 1, **dadurch gekennzeichnet daß** das Arzneimittel als Wirkstoff Riboflavin und/oder FAD enthält.

3. Verwendung von Riboflavin nach Ansprüche 1 und 2, **dadurch gekennzeichnet daß** das Arzneimittel außer Riboflavin und/oder FAD, Niazin und/oder NAD und NADP enthält.

4. Verwendung von Riboflavin nach Ansprüche 1 bis 3, **gekennzeichnet** daß das Arzneimittel als therapeutische Hilfmitteln, weitere vitaminische Faktoren, solche als Karotenen, Flavonoïden, Azetylcoenzym A und die Vitaminen A, E, C, B6, B8, und B9, enthält.

5. Verwendung von Riboflavin nach einer das Ansprüche 1 bis 4, **dadurch gekennzeichnet daß** die vitaminische Faktoren als einer Base und/oder in der ursprunglichen Form vorliegen.

6. Verwendung von Riboflavin nach einem der Ansprüche 1 bis 4, **dadurch gekennzeichnet daß** die Arzeimitteln außerdem Lysozym enthalten.

7. Verwendung von Riboflavin nach einer der Ansprüche 1 bis 4, **dadurch gekennzeichnet daß** die Arzneimitteln Glutathion und N-Acetylcystein enthalten.

8. Verwendung von Riboflavin nach einer der Ansprüche 1 bis 4, **dadurch gekennzeichnet daß** die Arzneimitteln einen Salz des Baryums aus dem Sulfat und dem Titanat gewählt sind und/oder ein Derivat des Titans aus dem Titan Dioxid, dem Titan Chlorid und dem Titan-Schlimmer, gewählt, sind enthalten.

9. Verwendung von Riboflavin nach einer der vorstehenden Ansprüche, **dadurch gekennzeichnet daß** dieses unter die verschiedenen galenikalen Formen die für den Schutz der genitalen Bereichs geeignet ist, angeboten wird.

10. Verwendung von Riboflavin nach einer der vorstehenden Ansprüche, **dadurch gekennzeichnet daß** diese unter die verschiedenen galenikalen Formen die für den Schutz der rektalen Schleimhautes geeignet sind, angeboten wird.

## Claims

1. The use of riboflavin for the achievement of an antiviral medicine with local action intended for the treatment of genital and rectal mucosae.

2. The use of riboflavin according to claim 1, wherein the medicine contains as the active ingredient, riboflavin and/or FAD.

3. The use of riboflavin according to claims 1 and 2, wherein the medicine contains supplementary to riboflavin and/or to FAD, niacin and/or NAD and NADP.

4. The use of riboflavin according to claim 1 to 3, wherein the medicine contains as therapeutic adjuvants, other vitaminic factors such as carotens, flavonoïds, acetyl coenzyme A, and the vitamins A, E, C, B6, B8 and B9.

5. The use of riboflavin according to any one of claim 1 to 4, wherein the vitaminic factors are in the form of a base and/or in their native form.

6. The use of riboflavin according to any one of claims 1 to 4, wherein the medicine further contain lysozym.

7. The use of riboflavin according to any one of claims 1 to 4, wherein the medicines contain glutathion and N-acetyl cystein.

8. The use of riboflavin according to one of claims 1 to 4, wherein the medicines contain a baryum salt selected among the sulfate and the titanate and/or a derivative of titanium selected among titanium dioxide, titanium chloride and micatitan.

9. The use of riboflavin according to one of the preceding claims, wherein it is offered in the various galenical forms suitable for the protection or the genital area.

10. The use of riboflavin according to one of the preceding claims wherein it is offered in the various galenical forms suitable for the protection of the rectal mucosa.
